# FASCICULE DE BREVET EUROPEEN

(11) **EP 1 365 795 B1**
(45) Date de publication et mention de la délivrance du brevet: **19.05.2010**
(21) Numéro de dépôt: 02702463.7
(22) Date de dépôt: 07.02.2002
(51) Int. Cl.: A61K 38/19, A61P 19/00

(54) **UTILISATION DU G-CSF COMME TRAITEMENT ADJUVANT DANS LA RECONSTRUCTION DE TISSUS CONJONCTIFS**
VERWENDUNG VON G-CSF ALS ZUSÄTZLICHE BEHANDLUNG IN DIE WIEDERHERSTELLUNG VON BINDEGEWEBE
USE OF THE G-CSF AS ADDITIONAL TREATMENT IN THE RECONSTRUCTION OF CONNECTIVE TISSUE

(30) Priorité: 22.02.2001 FR 0102406
(43) Date de publication de la demande: 03.12.2003
(73) Titulaire: Pourquier, Didier, 34070 Montpellier (FR); Moukoko, Didier, 34980 Saint Gely du Fesc (FR)
(72) Inventeur: Pourquier, Didier, 34070 Montpellier (FR); Moukoko, Didier, 34980 Saint Gely du Fesc (FR)
(74) Mandataire: Ahner, Francis
(86) Numéro de dépôt international: PCT/FR2002/000480
(87) Numéro de publication internationale: WO 2002/066051

(56) Documents cités:
- WO-A-98/51317
- WO-A-99/01145
- Y. TAKAMATSU ET AL.: "Osteoclast-mediated bone resorption is stimulated during short-term administration of granulocyte colony-stimulating factor but is not responsible for hematopoietic progenitor cell mobilization." BLOOD., vol. 92, no. 9, 1 novembre 1998 (1998-11-01), pages 3465-3473, XP002182647 W.B.SAUNDERS COMPAGNY, ORLANDO, FL., US ISSN: 0006-4971
- D. BONN: "The application of cell biology to broken bones." THE LANCET, vol. 353, 20 février 1999 (1999-02-20), page 650 XP002182648 LONDON, GB cité dans la demande

## Description

L'invention concerne une nouvelle application thérapeutique du G-CSF et plus particulièrement son utilisation pour la préparation d'un médicament utile en médecine humaine ou vétérinaire.

Le G-CSF (abréviation de "Granulocyte Colony-Stimulating Factor", c'est à dire "Facteur de Stimulation des Colonies de Granulocytes") est un facteur de croissance. Il correspond à une des classes de cytokines appelées facteurs de croissance hématopoïétique ou CSF (Colony-Stimulating Factors). Les CSF forment une famille de glycoprotéines ayant des fonctions capitales dans la formation des cellules sanguines. Le G-CSF stimule la production des cellules hématopoïétiques et de manière prédominante la production de polynucléaires. Le G-CSF est une glycoprotéines et le produit de l'expression d'un gène situé sur le chromosome 17. Il est produit par différentes cellules, telles que les fibroblastes, macrophages, cellules endothéliales, cellules épithéliales. Le G-CSF est détectable dans le sang, il peut être purifié à partir de surnageants de cultures de cellules tumorales humaines.

Le génie biologique permet aussi de produire un allotype humain de G-CSF, le G-CSF recombinant humain (rHuG-CSF). Il est à noter que le rHuG-CSF est efficace sur d'autres espèces animales (Gratwohl et col : Transplantation of G-CSF mobilized allogeneic peripheral blood stem cells in rabbits. Bone Marrow Transplant 1995 ;16(1):63-68).

Différents procédés de production du G-CSF sont décrits dans les publications de brevets suivantes: US 4 810 643, EP 0 169 566, WO 87 01132 et JP 15 327 384.

En France deux médicaments appartenant à la classe du G-CSF sont disponibles : le Neupogen® (r-metHuG-CSF, Filgrastime, commercialisé par Amgen/Produits Roche), et le Granocyte® (rHuG-CSF, Lénograstime, commercialisé par les Laboratoires Rhône-Poulenc Rorer), ces deux médicaments sont utilisés par voie d'injection sous-cutanée ou par perfusion intraveineuse, pour une administration systémique, à des doses généralement comprises entre 5 et 10 µg par kilogramme de poids corporel et par jour.

Les indications actuelles du G-CSF en thérapeutique humaine sont le traitement des neutropénies chroniques sévères, la réduction des neutropénies induites par les traitements chimiothérapiques anticancéreux myélotoxiques, la réduction des neutropénies induites par les thérapeutiques myélosuppressives (chimiothérapie ou radiothérapie) suivies de greffe de moelle dans le traitement des cancers ou des leucémies, la mobilisation des cellules souches hématopoïétiques pour constitution d'un greffon en vue d'une greffe de moelle (autogreffe ou allogreffe).

Il est à souligner que, dans cette dernière utilisation, l'objectif du traitement est de faire sortir de la moelle osseuse les cellules souches hématopoïétiques et de les faire passer dans le sang circulant. Ces cellules souches sont alors recueillies par cytaphérèses successives et vont constituer le greffon. Il s'agit donc de mobiliser au maximum vers le sang circulant ce contingent de cellules souches hématopoïétiques qui, à l'état normal, se trouvent en quantité très réduite dans le sang circulant, ce qui ne permet pas de constituer un greffon de qualité suffisante.

Cette technique de recueil du greffon par cytaphérèse après mobilisation des cellules souches est venue remplacer avantageusement le recueil direct de la moelle osseuse par cytoponction qui nécessite une anesthésie du patient et de multiples ponctions de moelle. Le greffon ainsi constitué est ensuite transfusé au patient, dont la propre moelle a été détruite par la chimiothérapie ou la radiothérapie ; il constitue alors la nouvelle source de production des cellules sanguines.

Le G-CSF est utilisé de manière quotidienne pour ces différentes indications. Son innocuité largement reconnue permet de l'utiliser aussi chez des personnes en bonne santé pour constituer des greffons de moelle dans le cadre des allogreffes.

Il convient de rappeler que la moelle osseuse, répartie dans les différents os de l'organisme, est le lieu de production des cellules sanguines matures (érythrocytes, plaquettes, polynucléaires, monocytes, lymphocytes). La production de ces cellules sanguines s'effectue à partir de la multiplication et de la différenciation d'une population de cellules souches pluripotentes (Cellule Souche Hématopoïétique, en abréviation "CSH"). Ces dernières représentent quantitativement une fraction très réduite de la population cellulaire de la moelle osseuse. Elles sont classiquement caractérisées par l'expression d'un marqueur cellulaire appelé CD34 (cluster de différenciation 34).

Cependant il a été décrit un autre type de cellules souches de la moelle osseuse (Pittenger et col : Multilineage potential of human mesenchymal stem cells. Science 1999 ; 284 : 143-147; Dennis et col : A quadripotential mesenchymal progenitor cell isolated from the marrow of an adult mouse. J Bone Miner Res 1999 ; 14(5): 700-709). Il s'agit d'une population de cellules capable d'une voie de différenciation conjonctive ; ces cellules souches sont appelées Cellules Souches Mésenchymateuses (en abréviation "CSM"). Cette population de cellules souches constitue, elle aussi, à l'état normal un contingent très réduit des éléments cellulaires de la moelle osseuse mais présente la capacité de se différencier dans de multiples directions de nature conjonctive (os, cartilage, fibrotendon, muscle, graisse). L'existence d'un précurseur commun (cellule souche CD34 négative) à l'ensemble des lignées hématopoïétiques et conjonctives est actuellement discutée (Seshi et col: Human bone marrow stromal cell: coexpression of markers specific for multiple mesenchymal stem lineage. Blood Cell Mol Dis 2000(3) : 234-246 ; Lange et col : Hematopoietic reconstruction of syngenic mice with a peripheral blood-derived, monoclonal CD34-, Sca-1+, Thy1(low), c-kit+ stem cell ligne. J Hematother Stem Cell Res. 1999 ; 8(4): 335-342).

Cette population de cellules souches est l'objet actuellement d'une activité de recherche intense, en particulier dans le domaine de la chirurgie plastique, de la réparation osseuse et de l'ostéoporose, ceci dans le cadre des domaines émergents de la médecine que sont l'ingénierie tissulaire et la thérapie cellulaire. Cependant, cette population de CSM constituant au départ une quantité de cellules particulièrement faible, les techniques utilisées font appel à un passage in vitro. Pour résumer, on recueille la moelle osseuse, puis in vitro on sépare le contingent des cellules souches mésenchymateuses du reste des cellules de la moelle, et ces cellules sont ensuite cultivées et multipliées toujours in vitro. Eventuellement elles sont aussi poussées artificiellement dans le sens d'une différenciation spécifique. Par la suite elles sont réinjectées ou réimplantées dans un site anatomique où elles contribuent à la reconstitution d'un tissu conjonctif délabré, qui peut être un os (Bruder et col : Bone regeneration by implantation of purified culture-expended human mesenchymal stem cells. J Ortho Res 1998 ; 16(2) : 155-162) ou bien un tendon (Awad et col : Autologous mesenchymal stem cell-mediated repair of tendon. Tissue Eng 1999 ; 5(3) : 267-277 ; Butler et col : Perspectives on cell and collagen composites for tendon repair. Clin Orthop 1999 ; 367 Suppl : S 324-332).

Ces techniques nécessitent cependant une collaboration avec des laboratoires très spécialisés et l'obtention d'un greffon conjonctif de qualité n'est pas toujours assurée (Solchaga et col : High variability in rabbit bone marrow-derived mesenchymal cell preparation. Cell Transplant 1999 ; 8(5): 511-519). La prolifération et la différenciation des CSM est induite par la Bone Morphogenetic Protein-2 {BMP-2} (Fromigue et col : Bone morphogenetic protein 2 and transforming growth factor-beta 2 interact to modulate human bone marrow stromal cell proliferation and differenciation. J Cell Biochem. 1998; 68(4) : 411-426), mais aussi les Transforming Growth Factor beta 1 (Andrades et col : A recombinant TGF-beta 1 fusion protein with collagen-binding domain promotes migration, growth, and differentiation of bone marrow mesenchymal cells. Exp Cell Res 1999 ; 250(2) : 485-498 )

Dans le domaine de la réparation des fractures osseuses, le processus de réparation osseuse après fracture d'un os est classiquement décrit en deux grandes phases. La première phase est une période d'union comportant successivement une période d'hématome, une période de prolifération cellulaire de cellules mésenchymateuses immatures à partir du périoste et des tissus conjonctifs périosseux, une période de constitution d'un cal mou où les cellules mésenchymateuses mais aussi des fibroblastes et des néo-vaisseaux envahissent l'espace du foyer de fracture, et une période de cal primaire où les cellules mésenchymateuses transformées dans un sens ostéocartilagineux constituent un massif tissulaire de résistance mécanique progressivement renforcée entre les deux extrémités osseuses. La deuxième phase est une période de remodelage, où l'os primaire se transforme en os tissé mature dont la forme finale s'adapte aux exigences des fonctions mécaniques du segment osseux concerné. Dans le cas des os longs la cavité médullaire est reconstituée ainsi qu'une corticale.

De nombreux médiateurs biochimiques sont impliqués dans le processus de réparation osseuse (Millis : Bone and non-bone derived growth factors and effects on bone healing. Vet Clin North Am Small Anim Pract 1999 ; 29(5) : 1221-1246). Ils peuvent agir de façon endocrine, paracrine ou autocrine. Les FGF (fibroblast growth factor), EGF (epidermal growth factor), PDGF (platelet-derived growth factor), IGF (insuline-like growth factor) I et II, TGF béta (transforming growth factor béta), les BMP (Bone Morphogenetic Proteins), les Prostaglandines, l'Ostéoglycine (osteo inductive factor) et l'Hormone de Croissance (GH) sont impliqués à différents niveaux.

Le traitement de base des fractures osseuses consiste essentiellement en la stabilisation du foyer de fracture. Cette stabilisation peut être obtenue par une contention du segment osseux dans le cadre d'un traitement orthopédique ou par ostéosynthèse si le recours à la chirurgie est nécessaire. Une prévention des infections et des accidents thromboemboliques est aussi nécessaire.

Il est à noter qu'il n'existe pas à l'heure actuelle de traitement médicamenteux par voie générale utilisé dans la pratique médico-chirurgicale quotidienne et capable d'améliorer la réparation des fractures osseuses, même si certains médicaments comme la L-Dopa ou des peptides de type FGF sont évalués par certains auteurs.

Des systèmes de délivrance locale, sur le foyer de réparation osseuse, de facteurs de croissance destinés à améliorer le processus de reconstruction osseuse sont aussi évalués par certains auteurs. Différentes molécules ont été utilisées : le TGF-béta (transforming growth factor béta), le FGF (fibroblast growth factor) les BMP, les BMP-2 et BMP-7 en particulier (Bonn : The application of cell biology to broken bones. Lancet 1999 ; 353 (9153) : 650). Des molécules telles que les héparanes sulfates semblent aussi prometteuses (Lafont et col: RGTA11, a new healing agent, triggers developmental events during healing craniotomy defects in adult rats. Growth Factors 1998 ; 16(1) : 23-38).

Par ailleurs, il a été développé un modèle de chirurgie expérimentale de construction ostéocartilagineuse par transfert de lambeau de périoste (Thèse de Docteur en Médecine D. Moukoko, Faculté de Médecine, Université Montpellier I 1996: Etude Expérimentale de l'ostéogenèse des lambeaux périostés vascularisés ; Premier Congrès de recherche en Orthopédie Pédiatrique Toulouse 1997 Présentation : L'ostéogenèse des lambeaux périostés vascularisés, D Moukoko, A Dimeglio ; Congrès annuel de l'European Pediatric Orthopedic Society Heidelberg 1997, Présentation : The osteogenical properties of periosteal vascularized flaps, D Moukoko, A Dimeglio ; Deuxième Congrès de Recherche en Orthopédie Pédiatrique, Montpellier 1998, Présentation : Histogenèse précoce des lambeaux périostés vascularisés dans un environnement musculaire, D Moukoko, D Pourquier, A Dimeglio).

Ce modèle chirurgical déjà bien connu (Poussa et col : The osteogenic capacity of free periosteal and osteoperiosteal grafts. Acta Orthop Scand 1979 ; 50 : 491-499 ; Ritsilä et col: Periosteal and perichondral grafting in reconstructive surgery. Clin Orthop 1994 ; 302 : 259-265 ; Finley et col : Revascularized periosteal grafts - A new method to produce functional new bone without bone grafting. Plast Reconstr Surg 1978 ; 61(1) : 1-6 ; Rubak : Reconstruction of articular cartilage defects with free periosteal grafts. Acta Orthop Scand 1982 ; 53 : 175 ; Masquelet et col : Vascularized periosteal grafts. Anatomic description, experimental study, preliminary report of clinical experience. Rev Chir Orthop Réparatrice Appar Mot 1988 ; 74 Suppl 2 : 240-243 ; van den Wildenberg et col : Free revascularised periosteum transplantation : an experimental study. Br Plast Surg 1984 ; 37(2) : 226-235 ; O'Driscoll et col : Durability of regenerated articular cartilage produced by free autogenous periosteal grafts in major full-thickness defects on joint surfaces under the influence of continuous passive motion. A follow-up report at one year. J Bone Joint Surg Am 1988 ; 70(4) : 595-606 ) dans le domaine de la reconstruction de cartilage articulaire ou de la reconstruction osseuse, a permis dans les travaux des Demandeurs de produire de l'os sur le site correspondant à la zone d'implantation du lambeau de périoste. Le transfert de lambeaux de périchondre permet une production cartilagineuse (Diaz-Flores et col : Growth of two types of cartilage after implantation of free autogenic perichondrial grafts. Clin Orthop 1988 Sep ; (234) : 267-279).

La procédure opératoire était la suivante :

Incision longitudinale de la face interne de la jambe. Exposition du muscle jambier antérieur, incision de son aponévrose longitudinalement à 5 mm de la crête tibiale antérieure. Mise en place de fils tracteurs évitant les manipulations du lambeau de périoste, incision du périoste longitudinalement, sur la face latérale de la diaphyse tibiale. Décollement du périoste, de son incision vers la crête tibiale postéro-médiale. Incision du périoste le long de la crête tibiale postéro-médiale (largeur du lambeau 10 mm). Incisions transversales, proximale et distale, libérant le lambeau sur 30 mm de long. Décollement du muscle fléchisseur des orteils, dont la vascularisation provient d'un pédicule différent. Le lambeau reste vascularisé sur le pédicule saphène par l'intermédiaire du fascia externe de jambe, de l'axe saphène à la crête tibiale antérieure, agissant comme lame porte-vaisseaux. Ainsi individualisé, le lambeau peut être positionné en situation ectopique ou orthotopique.

Une variante plus complexe de ce modèle permettait de transférer le lambeau vascularisé à proximité des structures osseuses du tibia et du fémur et de l'articulation du genou. Ce modèle a été utilisé à la fois sur le Lapin et sur le Mouton.

Le sacrifice de différents groupes d'animaux dans des délais post opératoires de longueurs croissantes, permettait d'étudier les étapes successives de la formation du cartilage et de l'os. Après le sacrifice des animaux, l'ensemble des tissus correspondant à l'os néoformé et aux autres tissus environnants était prélevé et étudié par histologie.

La néoproduction montrait, à l'étude histologique des différents délais, une séquence de prolifération cellulaire, différenciation cellulaire et tissulaire ; puis maturation des tissus néoformés. Cette séquence comprenait une phase de prolifération cellulaire d'un mésenchyme peu différencié, une phase de maturation de ce mésenchyme en un tissu cartilagineux, une phase d'ossification de cette matrice cartilagineuse par le biais d'un front d'ossification, une phase d'ossification complète de cet os sur le mode d'un os primaire, une phase de maturation de cet os primaire en un os secondaire de type tissé, la constitution d'une cavité médullaire remplie de moelle osseuse. L'ensemble réalisait finalement l'équivalent d'un petit os long avec une cavité médullaire et une corticale.

L'ensemble de ces observations montrait aussi un parallélisme saisissant avec la séquence d'événements biologiques qui président à la fois à la construction du bourgeon de membre pendant l'embryogenèse, et avec les différentes phases de la reconstruction osseuse dans les cals des fractures osseuses, ces deux processus étant considérés à l'heure actuelle comme très proches au plan de la biologie moléculaire (Ferguson et col : Common molecular pathways in skeletal morphogenesis and repair : Ann N Y Acad Sci : 1998 ; 23 857 : 33-42).

Des expérimentations complémentaires utilisant des membranes semi-perméables (membranes perméables aux molécules et imperméables aux cellules) montraient aussi que le lambeau de périoste agissait sur les tissus environnant par le biais de la diffusion locale de molécules biochimiques diffusibles, constituant en fait une zone d'influence du lambeau de périoste dans les tissus environnants. Une étude récente dans le domaine de la biologie du périoste montre qu'un des médiateurs émis précocement dans le milieu est la BMP 2 (Bone Morphogenetic Protein 2) (Sanyal et col : Initial evidence for the involvement of bone morphogenetic protein-2 early during periosteal chondrogenesis. J Orthop Res 1999 17(6) : 926-934), confirmant encore la similitude entre les événements développés dans les fractures osseuses et les événements développés dans le modèle de reconstruction osseuse par lambeau de périoste. Du point de vue physiopathologique cette émission locale de médiateurs biochimiques est engendrée par "l'activation" du périoste, cette activation étant elle-même déclenchée par le décollement mécanique du périoste de son support osseux (un des éléments fondamentaux de la physiopathologie de la fracture osseuse). Il est à noter que la capacité ostéogénique du périoste "activé" par décollement est aussi d'observation courante en pratique clinique, par exemple en cas de décollement tumoral, infectieux ou hématique. Certaines études montrent que le décollement du périoste et d'une fine couche osseuse sous-périostée enclenchent le même processus (Poussa et col. The osteogenic capacity of free periosteal and osteoperiosteal grafts. Acta Orthop Scand 1979 ; 50 : 491-499 ; Sakai et col : Free vascularized thin corticoperiosteal graft. Plast Reconstr Surg 1991 ; 87(2) : 290-298).

Cependant, au sein de ce modèle, des éléments de connaissance nouveaux sont apparus en particulier grâce aux études histologiques. Le plus important est la contribution de cellules souches mésenchymateuses présentes dans la circulation sanguine au processus de reconstruction osseux.

Une forme de contribution des CSM médullaires est déjà connue dans le cadre de la formation du cal de fracture au cours de la réparation osseuse. Cependant elle est entendue comme une contribution de nature purement locale, la moelle osseuse située dans le trait de fracture contribuant à la reconstruction de l'os fracturé.

Certains arguments histologiques sont venus démontrer que cette contribution se fait aussi à partir de cellules souches mésenchymateuses présentes dans le sang circulant qui sont recrutées localement par les différents médiateurs biochimiques émis dans les tissus autour du lambeau de périoste.

En particulier autour de l'os néoformé, il est d'observation fréquente d'identifier dans les muscles au sein des septas inter-musculaires (qui sont, il faut le rappeler, des lames porte-vaisseaux) une population de cellules différenciées dans les différentes directions conjonctives (os, cartilage, fibrotendon, muscle) et dans un sens hématopoïétique.

La situation micro anatomique spécifique de ces cellules dans les lames porte-vaisseaux montre clairement qu'elles sont issues du courant sanguin et ont été recrutées localement par les facteurs de croissance émis dans les tissus environnant le lambeau de périoste. Bien que clairement différenciés dans un sens conjonctif, ces éléments n'avaient cependant pas été intégrés au bloc osseux néoformé (données non publiées).

Une autre observation régulière au sein du modèle est la présence de cellules conjonctives des différents types et de cellules hématopoïétiques en masse dans les cavités articulaires présentes dans la zone d'influence du lambeau.

Ces images indiquent clairement une sortie vers la cavité articulaire de ces cellules à partir des vaisseaux du revêtement synovial, la cavité articulaire constituant alors un réceptacle (données non publiées).

Une autre observation intéressante était l'observation de zones de différenciation massive dans un sens conjonctif (os, cartilage, muscle, fibrotendon) de cellules de la moelle osseuse dans la cavité médullaire des os situés dans la zone d'influence du lambeau de périoste. Cette dernière observation montrait une influence identique du lambeau de périoste sur la population des CSM, mais cette fois-ci sur le contingent de CSM médullaires non circulant.

L'ensemble de ces observations laisse supposer un mécanisme de "recrutement" local dans la zone d'influence du lambeau d'une population de cellules souches présente dans le sang circulant. Ces cellules, en sortant des vaisseaux au niveau de la zone d'influence du lambeau, sont mises à la disposition du processus de reconstruction tissulaire comme des "briques" contribuant à la construction d'un édifice.

Un autre argument important était la présence très régulière d'un contingent de cellules hématopoïétiques matures mélangées avec des éléments conjonctifs. Ce dernier aspect peut en effet indiquer que le recrutement local de cellules souches concerne soit des cellules souches circulantes particulièrement immatures et pluripotentes capables de se différencier dans les directions à la fois hématopoïétique et conjonctive, soit les deux populations cellulaires souches (CSH et CSM) circulantes.

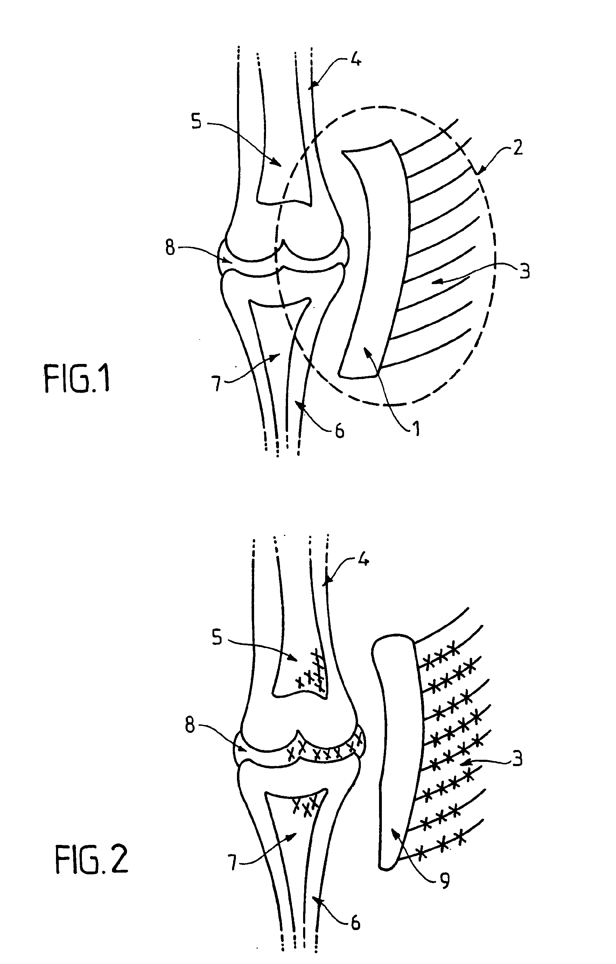

Les Figures 1 et 2 viennent illustrer les rapports du lambeau de périoste transféré et des différentes structures anatomiques intéressées.

La Figure 1 montre le lambeau de périoste 1 et sa zone d'influence 2 aux stades très précoces du modèle. La zone d'influence est entendue comme l'espace dans lequel la diffusion des facteurs de croissance et des différents médiateurs influence les tissus environnants. Sur cette figure, 3 désigne le muscle et les septas intermusculaires adjacents au lambeau de périoste 1. On a représenté en outre un fémur 4 et sa moelle osseuse 5 et un tibia 6 et sa moelle osseuse 7. Enfin 8 désigne la cavité articulaire comprise entre le fémur et le tibia.

La Figure 2 montre le résultat du transfert dans un délai plus long après le transfert du lambeau de périoste ; l'os néoformé 9 est apparu sur le site de transfert du lambeau, cependant que des foyers de différenciation conjonctive multidirectionnelle (représentés par des croix) sont apparus dans la moelle osseuse, les septas intermusculaires et la cavité articulaire situés dans la zone d'influence du lambeau de périoste.

En terme quantitatif, l'importance du recrutement local de CSM est cependant dépendante du nombre de cellules souches présentes dans le courant sanguin au niveau des vaisseaux situés dans la zone d'influence du lambeau. Comme déjà mentionné, cette population de cellules souches est, chez le sujet en bonne santé, quantitativement très réduite dans la moelle osseuse. Dans le sang, ces cellules souches sont identifiables à l'état normal (Huss et col : Evidence of peripheral blood-derived, plastic-adherent CD34(-/low) hematopoietic stem cell clones with mesenchymal stem cell characteristics. Stem Cell 2000 ; 18(4) : 252-260), mais aussi si le sujet a été traité par chimiothérapie myélotoxique suivie d'une mobilisation des cellules souches de la moelle osseuse par des facteurs de croissance de type G-CSF (Fernandez et col : Detection of stromal cells in peripheral blood progenitor cell collections from breast cancer patients. Bone marrow Transplant 1997 ; 20 : 265-271). Il est intéressant de noter que dans ce cas une augmentation de la quantité des CSM circulantes est corrélée à l'augmentation concomitante du nombre de CSH (CD 34 positives) circulantes, laissant supposer une influence des traitements sur les deux populations de cellules souches de la moelle osseuse (ou sur un précurseur commun) et sur leur mobilisation vers le sang circulant. Cependant la part respective du traitement chimiothérapique et du traitement par les facteurs de croissance dans l'augmentation du nombre de cellules souches circulantes reste difficile à préciser.

Les Demandeurs ont trouvé, de manière surprenante, une nouvelle application thérapeutique du G-CSF, tel que défini plus haut. Il a été découvert, à la suite de différents travaux et recherches, que le G-CSF pouvait aussi être utilisé dans la préparation d'un médicament pour le traitement adjuvant dans un processus de reconstruction des tissus conjonctifs chez un être vivant.

Par l'expression "tissus conjonctifs", on désigne principalement les os, tendons, fibrotendons, cartilages, muscles, etc, présents dans le corps d'un être vivant, en particulier chez l'homme et l'animal.

Sur la base des observations précédentes, les Demandeurs ont étudié l'influence de la stimulation de la moelle osseuse par le G-CSF sur le volume de la production osseuse dans le cadre de la formation d'os à partir de lambeaux de périoste vascularisés et transférés dans un environnement musculaire.

En effet le G-CSF possède un puissant pouvoir prolifératif sur la moelle osseuse. Bien que la lignée hématopoïétique (et en particulier la voie de différenciation vers les polynucléaires) soit surtout concernée par ce pouvoir prolifératif, il est fréquent que les facteurs de croissance possèdent un spectre d'action relativement large, et ce d'autant plus que le mode d'action de ces cytokines dans la mobilisation des cellules souches de la moelle n'est pas clair (Kronenwett et col : The role of cytokines and adhésion molécules for mobilization of peripheral blood stem cells. Stem Cells 2000 18(5) : 320-330). Les Demandeurs ont testé la capacité éventuelle du G-CSF à augmenter le volume de la production osseuse développée par un lambeau de périoste transféré dans un environnement musculaire (avec comme hypothèse physiologique que la prolifération et la mobilisation vers le sang circulant de cellules souches médullaires capables de se différencier dans un sens conjonctif permet d'augmenter la quantité de cellules souches circulantes disponibles dans la zone d'influence du lambeau de périoste, et donc la contribution éventuelle de ces cellules à la production d'os néoformé). Deux groupes d'animaux ont été constitués:

Un premier groupe (A) comportait 20 lapins adultes jeunes, et on réalisait sur ce groupe un transfert de lambeau de périoste sur le membre inférieur droit.

Un deuxième groupe (B) comportait un nombre identique d'animaux de même âge et de même poids et on réalisait le même transfert de lambeau de périoste. Cependant, la veille de l'intervention chirurgicale, le jour de l'intervention et les trois jours suivant l'intervention on procédait à une injection de rmetHuG-CSF (Neupogen® {filgrastime} Amgen Inc Califomie USA/ Produits Roche France) par voie sous cutanée à la dose de 10 µg (1MU) par kilogramme de poids corporel. Afin de travailler véritablement "en aveugle" et dans le but de ne pas influencer la qualité du geste chirurgical réalisé dans chaque groupe le chirurgien ignorait si l'animal opéré appartenait au groupe A ou au groupe B.

Les deux groupes d'animaux étaient ensuite laissés sans autre traitement hormis l'apport en eau et nourriture nécessaire aux besoins vitaux. Quinze jours suivant la date de l'intervention les animaux étaient sacrifiés et la néo-production osseuse extraite de son environnement musculaire. On évaluait ensuite le volume de l'os. L'os néoformé présentant une forme grossièrement cylindrique, on réalisait une mesure de la longueur et des mesures du diamètre maximum dans la partie médiane et aux deux extrémités du cylindre osseux afin d'obtenir un diamètre moyen. Le volume de l'os néoformé était calculé comme le volume d'un cylindre en fonction du diamètre moyen et de la longueur.

Le groupe A montrait un volume moyen d'os de 189,5 mm3, le groupe B un volume moyen d'os de 853,9 mm3 ; la longueur moyenne était dans le groupe A de 28,65 millimètres, dans le groupe B de 34,9 millimètres ; le diamètre moyen était dans le groupe A de 2,29 millimètres, dans le groupe B de 5,57 millimètres. La différence entre le groupe A et le groupe B était statistiquement significative par le test T de Student (volume A, diamètre A et longueur A inférieurs respectivement aux volume B, diamètre B et longueur B ; p inférieur à 0,00001, alpha= 5%), à la fois pour la longueur, pour le volume et pour le diamètre du cylindre d'os néoformé.

L'ensemble de ces résultats (résultats non publiés) est venu montrer que le G-CSF augmente la quantité d'os néoformé par un modèle in vivo de construction osseuse connu pour présenter une séquence d'événements biologiques identique au processus de réparation des fractures. De nombreux arguments tendent à montrer que cette augmentation pourrait être due à une augmentation du nombre de CSM présentes dans le sang circulant. Au cours des processus de construction osseuse à partir du lambeau de périoste ou dans le cadre d'une fracture osseuse ces éléments sont recrutés localement par les facteurs de croissance et différents médiateurs émis dans les tissus. Les CSM sortent des vaisseaux au niveau du foyer de construction osseuse et contribuent quantitativement à la constitution du cartilage et de l'os néoformé mais peuvent probablement contribuer aussi à la reconstitution des différents tissus conjonctifs de type fibrotendon ou muscle.

L'invention concerne donc plus particulièrement l'utilisation du G-CSF dans la préparation d'un médicament pour le traitement adjuvant dans des processus de reconstruction de tissus conjonctifs. Il trouve ainsi une application nouvelle et intéressante dans les thérapeutiques chirurgicales et/ou médicales utilisées dans le cadre de la pathologie de l'appareil locomoteur.

Conformément à l'invention, le G-CSF peut être utilisé comme médicament dans le traitement des fractures osseuses, ostéocartilagineuses, des pseudo-arthroses, des retards de consolidation des fractures osseuses, des lésions cartilagineuses, ou encore des ruptures ou sections de tendons et ligaments et des lésions des muscles. Il peut aussi être utilisé comme médicament dans le traitement d'allongement des membres, ou bien encore comme médicament dans le traitement associant un geste chirurgical de reconstruction osseuse ou cartilagineuse par transfert de lambeau de périoste, et de manière générale dans la chirurgie de régénération tissulaire des membres et de l'appareil locomoteur. Dans ces différentes applications le G-CSF est destiné à l'administration par voie générale.

Il entre également dans le cadre de l'invention d'utiliser le G-CSF comme médicament destiné à l'administration par voie générale et de le combiner à au moins un autre facteur destiné à l'administration locale ou par voie générale. L'expression "administration locale" signifie que l'administration du principe thérapeutique s'effectue sur le site anatomique où l'on souhaite reconstruire l'os ou le cartilage.

Cet autre facteur est avantageusement choisi parmi l'un au moins des facteurs suivants : BMP (Bone Morphogenetic Protein), SCF (stem cell factor), FGF (fibroblast growth factor), EGF (epidermal growth factor), IGF (insuline-like growth factor), Insuline, KGF (keratinocyte growth factor), TGF (transforming growth factor), Interféron, Interleukine, VEGF (vascular endothélial growth factor), TNF (tumor necrosing factor), GDNF (glial cell ligne-derived neurotrophic factor), NGF (neurotrophin nerve growth factor), GM-CSF (granulocytemacrophage colony stimulating factor), HGF (hepatocyte growth factor), Erythropoïetine, PDGF (platelet-derived growth factor), Héparane Sulfate, Prostaglandines, Ostéoglycine (osteoinductive factor), BCDF (B cell differentiation factor), GDF-5 (growth and differentiation factor-5), Hormone de Croissance ; M-CSF (macrophage colony stimulating factor) ; ou tout facteur de croissance connu d'origine humaine ou animale, d'extraction ou recombinant.

Pour la mise en oeuvre de l'invention, le G-CSF est avantageusement un recombinant humain (pour la médecine humaine), ou la Filgrastime, ou bien encore la Lénograstime.

Le G-CSF utilisé sera de préférence d'un allotype humain dans le cadre du traitement d'une personne humaine. Le dosage utilisé sera généralement de 0,1 à 1000 µg (0,01 à 100 MU) par kilogramme de poids corporel et par jour. De manière préférentielle on utilisera une dose de 5 à 10 µg par kilogramme de poids corporel et par jour.

Le principe de l'invention veut que le G-CSF soit administré par voie générale, ce qui signifie que le G-CSF entre dans la circulation sanguine générale. Le mode de délivrance pour obtenir une administration par voie générale peut comprendre un mode par injection intravasculaire directe, un mode par injection sous-cutanée, par injection intramusculaire, par injection intra-articulaire, une délivrance par voie digestive, injection intrapéritonéale, délivrance transpulmonaire, transcutanée, transbuccale, transnasale, transrectale, transconjonctivale, intrarachidienne.

Le principe biologique de l'invention est de mobiliser les CSM (ou le précurseur commun des CSM et des CSH) de la moelle osseuse vers le sang circulant. Cette augmentation du nombre de cellules souches dans la circulation sanguine permet aux médiateurs biochimiques émis localement dans les fractures osseuses de recruter dans le foyer de fracture une quantité de CSM supérieure à la quantité de CSM que la nature aurait pu fournir à l'état physiologique.

Le même mécanisme adjuvant est proposé pour la réparation des tendons ou des ligaments, car l'apport direct sur le site de réparation de cellules souches mésenchymateuses contribue à une amélioration significative du processus de réparation (amélioration des performances mécaniques), (Awad et col : Autologous mesenchymal stem cell-mediated repair of tendon. Tissue Eng 1999 ; 5(3) : 267-277 ; Butler et col : Perspectives on cell and collagen composites for tendon repair. Clin Orthop; 367 Suppl : S 324-332).

L'invention sera maintenant décrite en référence à des exemples.

### Exemple N°1: Traitement des pseudarthroses

La pseudarthrose est un défaut d'union spontané et définitif des segments osseux dans les suites d'une fracture osseuse ou d'une ostéotomie. Une fibrose s'interpose généralement entre les extrémités des deux segments osseux. Le traitement classique consiste en l'excision de la fibrose d'interposition, on peut aussi apporter localement de l'os par une greffe osseuse. Un geste chirurgical complémentaire et plus spécifique du traitement des pseudarthroses consiste à réaliser une décortication sur la circonférence des os à l'extrémité des segments osseux désunis. Dans le cas de la présente invention on injectera au patient du G-CSF (Neupogen® {Filgrastime}) à la dose de 10 µg (1MU) par kilogramme de poids corporel, 48 heures et 24 heures avant l'intervention, le jour de l'intervention, 24 heures et 48 heures après l'intervention. Le geste chirurgical de décortication (qui emporte une fine lamelle d'os cortical tout en gardant ce dernier attaché au périoste vascularisé qui le recouvre) réalise en fait l'équivalent d'une activation du périoste tout à fait comparable à celle qui est obtenue dans la chirurgie de transfert des lambeaux de périoste ou par le décollement du périoste dans les fractures des os. Le processus de reconstruction osseuse se trouve relancé et le traitement par le G-CSF vient augmenter encore la performance du geste chirurgical.

### Exemple N°2: Traitement d'une fracture osseuse grave

Il s'agit du traitement de fractures telles que, par exemple, fracture à fragments multiples, fracture ouverte, fracture associée à une perte de substance osseuse et/ou à un délabrement musculaire. Dans cette situation la prise en charge thérapeutique se fait dans l'urgence. Le traitement comportera la prise en charge chirurgicale classique. Une première dose de 10 µg (1 MU) par kilogramme de poids corporel de G-CSF (Neupogen®, Filgrastime) sera injectée par voie sous cutanée au patient dès son admission à l'hôpital. La même dose sera injectée de manière quotidienne pendant les quatre jours suivants. Le G-CSF permettra une colonisation maximale de l'hématome fracturaire par des CSM, induisant la reconstruction d'un cal de fracture de bonne qualité.

### Exemple N°3: Dans le cadre d'un geste chirurgical de reconstruction cartilagineuse par transfert de lambeau de périoste, pour resurfaçage d'un cartilage articulaire

Un transfert de lambeau de périoste vascularisé dans un environnement musculaire est réalisé, on ré-intervient deux jours plus tard sur le même site opératoire, et on prélève le tissu développé dans le site opératoire (matériel constitué de cellules souches mésenchymateuses). Ce matériel est ensuite placé dans l'articulation qui nécessite un resurfaçage où il va régénérer le cartilage lésé. Dans ce cas une dose 10 µg de G-CSF (Neupogen®, Filgrastime) par kilogramme de poids corporel sera injectée par voie sous-cutanée quotidiennement pendant les deux jours qui précédent l'intervention, le jour de l'intervention, les deux jours qui suivent l'intervention. Le principe thérapeutique évite le passage in vitro utilisé dans les méthodes d'ingénierie tissulaire ou de thérapie cellulaire, l'utilisation de GCSF augmente le nombre de cellules mésenchymateuses présentes dans le site opératoire et utilisable pour le resurfaçage articulaire.

### Exemple N°4 : Dans le cadre d'un geste chirurgical de reconstruction d'os par transfert de lambeau périosté pour combler une perte de substance massive.

En cas de perte de substance osseuse massive, les sources d'os de substitution récupérable dans l'organisme sont assez limitées. Pour l'essentiel il s'agit des greffes d'os à partir des crêtes iliaques, et des transferts de Péroné pour la chirurgie de la jambe. D'autre part, on considère que les greffes osseuses fournissent de l'os mort. Le geste chirurgical consistera en un transfert de lambeau de périoste vascularisé ; on laissera le lambeau évoluer en un os assez mature (par exemple pendant trois semaines), on interviendra alors de nouveau sur le site opératoire pour récupérer cet os, on prendra soin de récupérer le pédicule vasculaire source du lambeau et de l'os néoformé. Cet ensemble sera transféré sur le site anatomique de la perte de substance osseuse, et on procédera à une réimplantation vasculaire du pédicule source sur un axe artério-veineux adapté. Dans ce cas une dose de 10 µg de G-CSF (Neupogen®, Filgrastime) par kilogramme de poids corporel sera injectée par voie sous-cutanée quotidiennement pendant les deux jours qui précédent la première intervention, le jour de la première intervention, les deux jours qui suivent la première intervention. Le transfert et la réimplantation du pédicule vasculaire évitent le transfert d'un os mort, l'utilisation du G-CSF vient augmenter le volume de l'os néoformé transféré et donc augmenter les performances du procédé.

### Exemple N°5 : Dans le cadre d'une chirurgie d'allongement des membres

Pour allonger un membre, le traitement actuel comporte une section du segment osseux qui doit être allongé puis la mise en place d'un système de fixateurs externes ou d'un dispositif centro-médullaire permettant un écartement progressif des segments osseux au fur et à mesure de la reconstruction du cal (la stimulation de l'ostéogénèse est maintenue par l'écartement permanent des deux segments osseux). Chez certains patients l'os néoformé est cependant d'un calibre nettement inférieur au calibre des segments osseux sus et sous jacents ; l'os néoformé est donc d'aspect grêle et de résistance mécanique réduite. On propose donc comme traitement adjuvant une dose quotidienne de 10 µg de G-CSF par voie-sous-cutanée tout au long de la procédure d'allongement.

### Exemple N°6 : Dans le cadre d'une chirurgie de réparation des tendons

En cas de rupture ou de section traumatique du tendon, le traitement classique consiste en une chirurgie immédiate avec suture des deux extrémités tendineuses, une immobilisation du tendon puis une remobilisation du tendon. Le traitement adjuvant comportera une première dose de 10 µg (1 MU) par kilogramme de poids corporel de G-CSF (Neupogen®, Filgrastime) qui sera injectée par voie sous-cutanée au patient dès son admission à l'hôpital. La même dose sera injectée de manière quotidienne pendant les quatre jours suivant l'intervention.

Bien entendu les exemples ci-dessus sont donnés seulement à titre illustratif et n'entendent pas limiter la portée de l'invention

## Revendications

1. Utilisation du G-CSF (Facteur de Stimulation des Colonies de Granulocytes) pour la préparation d'un médicament utile comme traitement adjuvant dans un processus de reconstruction de tissus conjonctifs *in vivo* chez un être vivant.

2. Utilisation selon la revendication 1, **caractérisée en ce que** le G-CSF est utilisé comme médicament dans le traitement des fractures osseuses, ostéocartilagineuses, des pseudoarthroses, des retards de consolidation des fractures osseuses, des lésions cartilagineuses, ou encore des ruptures ou sections de tendons ou ligaments et des lésions des muscles et est destiné à l'administration par voie générale.

3. Utilisation selon la revendication 1, **caractérisée en ce que** le G-CSF est utilisé comme médicament dans le traitement d'allongement des membres et est destiné à l'administration par voie générale.

4. Utilisation selon la revendication 1, **caractérisée en ce que** le G-CSF est utilisé comme médicament dans le traitement associant un geste chirurgical de reconstruction osseuse ou cartilagineuse par transfert de lambeau de périoste, et de manière générale dans la chirurgie de régénération tissulaire des membres et de l'appareil locomoteur, et est destiné à l'administration par voie générale.

5. Utilisation selon l'une des revendications 1 à 4, **caractérisée en ce que** le G-CSF est utilisé comme médicament destiné à l'administration par voie générale et est combiné à au moins un autre facteur destiné à l'administration locale ou par voie générale.

6. Utilisation selon la revendication 5, **caractérisée en ce que** cet autre facteur est choisi parmi l'un au moins des facteurs suivants : BMP (Bone Morphogenetic Protein), SCF (stem cell factor), FGF (fibroblast growth factor), EGF (epidermal growth factor), IGF (insuline-like growth factor), Insuline, KGF (keratinocyte growth factor), TGF (transforming growth factor), Interféron, Interleukine, VEGF (vascular endothélial growth factor), TNF (tumor necrosing factor), GDNF (glial cell ligne-derived neurotrophic factor), NGF (neurotrophin nerve growth factor), GM-CSF (granulocytemacrophage colony stimulating factor), HGF (hepatocyte growth factor), Erythropoïétine, PDGF (platelet-derived growth factor), Héparane Sulfate, Prostaglandines, Ostéoglycine (osteoinductive factor), BCDF (B cell differentiation factor), GDF-5 (growth and differentiation factor-5), Hormone de Croissance ; M-CSF (macrophage colony stimulating factor) ; ou tout facteur de croissance connu d'origine humaine ou animale, d'extraction ou recombinant.

7. Utilisation selon l'une des revendications 1 à 6, **caractérisée en ce que** le G-CSF est un recombinant humain.

8. Utilisation selon l'une des revendications 1 à 6, **caractérisée en ce que** le G-CSF est la Filgrastime.

9. Utilisation selon l'une des revendications 1 à 6, **caractérisée en ce que** le G-CSF est la Lénograstime.

10. Utilisation selon l'une des revendications 1 à 9, **caractérisée en ce que** le médicament est utilisé avec un dosage en G-CSF de 0, 1 à 1000 µg (0,01 à 100 MU), de préférence de 5 à 10 µg, par kilogramme de poids corporel et par jour.

11. Utilisation selon l'une des revendications 1 à 9, **caractérisée en ce que** le médicament est utilisé en médecine humaine.

12. Utilisation selon l'une des revendications 1 à 10, **caractérisée en ce que** le médicament est utilisé en médecine vétérinaire.

## Claims

1. The use of G-CSF (Granulocyte colony Stimulation Factor) for preparing a drug useful as an adjuvant treatment in a process for reconstructing in vivo connective tissues in a living being.

2. The use according to claim 1, **characterized in that** G-CSF is used as a drug in the treatment of osteocartilaginous, bone fractures, pseudo-arthroses, delays of consolidation of bone fractures, cartilaginous lesions, or further breakages or severances of tendons or ligaments and muscle lesions and is intended to be administrated via a general route.

3. The use according to claim 1, **characterized in that** G-CSF is used as a drug in the treatment of extension of limbs and is intended to be administrated via a general route.

4. The use according to claims 1, **characterized in that** G-CSF is used as a drug in the treatment associating a cartilaginous or bone reconstruction surgical procedure by transfer of a periosteal graft, and generally in surgery for tissue regeneration of limbs and of the locomotor system, and is intended to be administrated via a general route.

5. The use according to any of claims 1 to 4, **characterized in that** G-CSF is used as a drug intended to be administrated via a general route and is combined with at least one other factor intended to be administrated locally or via a general route.

6. The use according to claims 5, **characterized in that** this other factor is selected from at least one of the following factors: BMP (Bone Morphogenetic Protein), SCF (stem cell factor), FGF (fibroblast growth factor), EGF (epidermal grow factor), IGF (insulin-like growth factor), insulin, KGF (keratinocyte growth factor), TGF (transforming growth factor), interferon, interleukin, VEGF (vascular endothelial growth factor), TNF (tumor necrosing factor), GDNF (glial cell line-derived neurotrophic factor), NGF (neurotrophin nerve growth factor), GM-CSF (granulocyte macrophage colony stimulating factor), HGF (hepatocyte growth factor), erythropoietin, PDGF (platelet-derived growth factor), heparane sulfate, prostaglandins, osteoglycin (osteoinductive factor), BCDF (B cell differentiation factor), GDF-5 (growth and differentiation factor-5), growth hormone; M-CSF (macrophage colony stimulating factor); or any known growth factor of human or animal origin, either extracted or recombinant.

7. The use according to any of claims 1 to 6, **characterized in that** G-CSF is a human recombinant.

8. The use according to any of claims 1 to 6, **characterized in that** G-CSF is Filgrastime.

9. The use according to any of claims 1 to 6, **characterized in that** G-CSF is Lenograstime.

10. The use according to any of claims 1 to 9, **characterized in that** the drug is used with a G-CSF dosage from 0.1 to 1,000 µg (0.01 to 100 MU), preferably from 5 to 10 µg, per kilogram of body weight and per day.

11. The use according to any of claims 1 to 9, **characterized in that** the drug is used in human medicine.

12. The use according to any of claims 1 to 10, **characterized in that** the drug is used in veterinary medicine.

## Patentansprüche

1. Verwendung des G-CSF (Granulocyten-Kolonie stimulierender Faktor) für die Zubereitung eines Arzneimittels, das als Zusatzbehandlung in einem in vivo-Rekonstruktionsprozess von Bindegeweben in einem Lebewesen nützlich ist.

2. Verwendung nach Anspruch 1, **dadurch gekennzeichnet, dass** der G-CSF als Arzneimittel bei der Behandlung von Knochen-, Knochen-Knorpel-Frakturen, Pseudoarthrosen, Verzögerungen bei der Konsolidierung von Knochenfrakturen, Knorpelverletzungen oder auch bei Rissen oder Durchtrennungen von Bändern oder Sehnen und Muskelverletzungen verwendet wird und zur Verabreichung auf systemischem Weg bestimmt ist.

3. Verwendung nach Anspruch 1, **dadurch gekennzeichnet, dass** der G-CSF als Arzneimittel bei der Behandlung zur Verlängerung der Gliedmaßen verwendet wird und zur Verabreichung auf systemischem Weg bestimmt ist.

4. Verwendung nach Anspruch 1, **dadurch gekennzeichnet, dass** der G-CSF als Arzneimittel bei der Behandlung in Kombination mit einer chirurgischen Tat zur Rekonstruktion von Knochen oder Knorpel durch Transfer von Knochenhautlappen verwendet wird und im allgemeinen in der Chirurgie zur Gewebsregenerierung der Gliedmaßen und des Bewegungsapparats und zur Verabreichung auf systemischem Weg bestimmt ist.

5. Verwendung nach einem der Ansprüche 1 bis 4, **dadurch gekennzeichnet, dass** der G-CSF als Arzneimittel verwendet wird, das zur Verabreichung auf systemischem Weg bestimmt ist und mit mindestens einem anderen Faktor kombiniert wird, der zur Verabreichung auf lokalem oder auf systemischem Weg bestimmt ist.

6. Verwendung nach Anspruch 5, **dadurch gekennzeichnet, dass** dieser Faktor mindestens aus einem der folgenden Faktoren ausgewählt ist: BMP (Bone Morphogenetic Protein), SCF (Stem Cell Factor), FGF (Fibroblast Growth Factor), EGF (Epidermal Growth Factor), IGF (Insulin-like Growth Factor), Insulin, KGF (Keratinocyte Growth Factor), TGF (Transforming Growth Factor), Interferon, Interleukin, VEGF (Vascular Endothelial Growth Factor), TNF (Tumor Necrosing Factor), GDNF (Glial Cell ligne-derived Neurotrophic Factor), NGF (Neurothophin nerve Growth Factor), GM-CSF (Granulocytemacrophage Colony Stimulating Factor), HGF (Hepatocyte Growth Factor), Erythropoietin, PDGF (Platelet-Derived Growth Factor), Heparansulfat, Prostaglandine, Osteogylcin (osteoinduktiver Faktor), BCDF (B-Cell Differentiation Factor), GDF-5 (Growth and Differentiation Factor-5), Wachstumshormon; M-CSF (Macrophage Colony Stimulating Factor) oder jedem bekannten Wachstumsfaktor menschlichen oder tierischen Ursprungs, Extraktions- oder rekombinanten Faktor.

7. Verwendung nach einem der Ansprüche 1 bis 6, **dadurch gekennzeichnet, dass** der G-CSF ein humanes Rekombinant ist.

8. Verwendung nach einem der Ansprüche 1 bis 6, **dadurch gekennzeichnet, dass** der G-CSF Filgrastim ist.

9. Verwendung nach einem der Ansprüche 1 bis 6, **dadurch gekennzeichnet, dass** der G-CSF Lenograstim ist.

10. Verwendung nach einem der Ansprüche 1 bis 9, **dadurch gekennzeichnet, dass** das Arzneimittel mit einer G-CSF-Dosierung von 0,1 bis 1000 µg (0,01 bis 100 MU), vorzugsweise von 5 bis 10 µg je Kilogramm Körpergewicht und je Tag, verwendet wird.

11. Verwendung nach einem der Ansprüche 1 bis 9, **dadurch gekennzeichnet, dass** das Arzneimittel in der Humanmedizin verwendet wird.

12. Verwendung nach einem der Ansprüche 1 bis 10, **dadurch gekennzeichnet, dass** das Arzneimittel in der Veterinärmedizin verwendet wird.
